# EUROPEAN PATENT SPECIFICATION

(11) **EP 3 405 117 B1**
(45) Date of publication and mention of the grant of the patent: **15.07.2026**
(21) Application number: 17741912.4
(22) Date of filing: 19.01.2017
(51) Int. Cl.: A61B 17/00, A61F 2/00, A61F 2/06, A61F 2/958, A61M 25/00, A61M 25/10

(54) **MEDICAL BALLOON WITH REINFORCEMENT STRUCTURE**
MEDIZINISCHER BALLON MIT VERSTÄRKUNGSSTRUKTUR
BALLONNET MÉDICAL DOTÉ DE STRUCTURE DE RENFORCEMENT

(30) Priority: 20.01.2016 US 201662280984 P
(43) Date of publication of application: 28.11.2018
(73) Proprietor: Boston Scientific Scimed, Inc., Maple Grove, MN 55311 (US)
(72) Inventor: KAO, Stephen, Saint Louis Missouri 63131 (US); LAGOE, Dan, Saint Louis Missouri 63131 (US); RECINELLA, Dan, Saint Louis Missouri 63131 (US); CHINUBHAI, Abha, Saint Louis Missouri 63131 (US); MARCY, Rodney, Saint Louis Missouri 63131 (US); MCCARTHY, Kim, Saint Louis Missouri 63131 (US); SOLANO, Scott, Saint Louis Missouri 63131 (US); NEGLEN, Peter, Saint Louis Missouri 63131 (US)
(74) Representative: Vossius & Partner Patentanwälte Rechtsanwälte mbB
(86) International application number: PCT/US2017/014109
(87) International publication number: WO 2017/127527

(56) References cited:
- WO-A1-96/40346
- US-A- 5 378 239
- US-A- 5 556 413
- US-A1- 2005 038 468
- US-A1- 2012 277 783
- US-A1- 2014 171 912
- US-B1- 6 585 926
- US-B2- 8 715 229

## Description

### CROSS-REFERENCE TO RELATED APPLICATIONS

This application claims the benefit of and priority to U.S. Provisional Application No. 62/280,984, filed January 20, 2016.

### BACKGROUND

Balloons may be used in a variety of medical procedures, including opening an area of the human body. During such procedures, an uninflated medical balloon is generally inserted into a body space and inflated by a user providing a pressure increase to the balloon. The balloon can subsequently expand, and the body space can thus be expanded and/or opened.

Chronic Venous Insufficiency (CVI) is a medical condition where veins cannot pump enough blood back to the heart. This is generally due to a higher blood pressure inside the leg veins. Other causes of CVI include deep vein thrombosis whereby a thrombus (blood clot) blocks portions of the veins and impedes blood flow to the heart.

In certain cases of CVI, a stent implementation can be performed to increase the blood flow through the veins. In such a procedure, elimination or correction of any obstructions prior to placement of a stent implant is necessary in order to obtain optimal clinical outcomes. Obstructions may include small amounts of "web" damage left in the vein, or scar tissue that exists and causes a loss of flow in the vein.

In a stent implantation procedure, a physician may attempt to break up fibrotic tissue such as scar tissue or webs using venoplasty, thrombolytics, or mechanical thrombectomy devices prior to stent implantation. The breaking up of the scar tissue may occur during a pre-dilatation procedure so as to ensure that unwanted or otherwise difficultycausing fibrous tissue is moved or removed before the stent is implanted. The process of breaking up scar tissue or webs can be a difficult procedure and often involves the use of high pressure.

Medical balloons with different types of reinforcement mechanisms are known. However, these medical balloons may not perform satisfactorily during high pressure operation, such that the balloon can fail at these high pressures, resulting in difficulty in removing fibrotic tissue that requires higher pressures for such a removal. Thus, using known medical balloons, the removal of the scar tissue or webs can be very time consuming and the scar tissue or webs may not be removed to an acceptable or expected level. Accordingly, it would be desirable to have a balloon operable at higher pressures, which may be used, for example, when breaking up fibrotic tissue to create an open lumen prior to placement of a stent.

US 5 378 239 A discloses a balloon arrangement comprising a balloon and an outer reinforcement structure.

### SUMMARY

The present application is directed to a balloon arrangement that includes a balloon layer having a base layer formed of polymer material, and a cage independent from the balloon layer, as defined in the independent claim.

The cage includes a monofilament and/or a multifilament material, and is adapted to come into contact with the balloon layer during pressurization. The cage in various embodiments is braided.

One or more embodiments of a balloon according to the present application may be able to break up unwanted fibrotic tissue and create an open lumen. This can be advantageously preformed as a pre-dilatation procedure and can allow for unwanted fibrotic tissue that may impinge on a stent to be inserted to be eliminated, thereby eliminating a possibility that such tissue would impinge on the stent to be inserted and limit or constrict its diameter.

### BRIEF DESCRIPTION OF THE DRAWINGS

Fig. 1 shows a balloon arrangement including a reinforcement structure according to an embodiment.
Fig. 2 shows the balloon arrangement in an expanded state according to an embodiment.
Fig. 3 shows the balloon arrangement in a compressed state according to an embodiment.
Fig. 4 shows the balloon arrangement coupled with an injection structure according to an embodiment.
Fig. 5 shows different components of a balloon arrangement in accordance with an embodiment.
Fig. 6 is a flow chart of a method for treating of tissue according to an embodiment.

### DETAILED DESCRIPTION OF EMBODIMENTS

In the following description, numerous details are set forth to provide an understanding of the present disclosure. However, it should be understood by those skilled in the art that the devices of the present disclosure may be practiced without these details and that numerous variations or modifications from the described embodiments may be possible.

At the outset, it should be noted that in the development of any such actual embodiment, numerous implementation-specific decisions may be made to achieve the developer's specific goals, such as compliance with system related and business related constraints, which will vary from one implementation to another. Moreover, it will be appreciated that such a development effort might be complex and time consuming but would nevertheless be a routine undertaking for those of ordinary skill in the art having the benefit of this disclosure. In addition, the device described herein can also comprise some components other than those cited. In the summary and this detailed description, each numerical value should be read once as modified by the term "about" (unless already expressly so modified), and then read again as not so modified unless otherwise indicated in context. Also, in the summary and this detailed description, it should be understood that a range listed or described as being useful, suitable, or the like, is intended to include support for any conceivable sub-range within the range at least because every point within the range, including the end points, is to be considered as having been stated. For example, "a range of from 1 to 10 is to be read as indicating each possible number along the continuum between about 1 and about 10. Furthermore, the subject matter of this application illustratively disclosed herein suitably may be practiced in the absence of any element(s) that are not specifically disclosed herein.

The following detailed description of certain embodiments will be better understood when read in conjunction with the appended drawings. To the extent that the figures illustrate diagrams of the different components of various embodiments, the illustrations are not necessarily Indicative of the division between hardware. Thus, for example, one or more of the components may be implemented in a single piece of hardware or multiple pieces of hardware. It should be understood that the various embodiments are not limited to the arrangements and instrumentality shown in the drawings.

As used herein, the terms "system," "subsystem", "unit," or "module" may include any combination of hardware and/or software system that operates to perform one or more functions. For example, a system, unit, or module may include a computer processor, controller, or other logic-based device that performs operations based on instructions stored on a tangible and non-transitory computer readable storage medium, such as a computer memory. Alternatively, a system, subsystem, unit, or module may include a hard-wired device that performs operations based on hard-wired logic of the device. The systems, subsystems, modules, or units shown in the attached figures may represent the hardware that operates based on software or hardwired instructions, the software that directs hardware to perform the operations, or a combination thereof.

As used herein, an element or step recited in the singular and proceeded with the word "a" or "an" should be understood as not excluding plural of said elements or steps, unless such exclusion is explicitly stated. Furthermore, references to "one embodiment" are not intended to be interpreted as excluding the existence of additional embodiments that also incorporate the recited features. Moreover, unless explicitly stated to the contrary, embodiments "comprising" or "having" an element or a plurality of elements having a particular property may include additional such elements not having that property.

The following definitions are provided in order to aid those skilled in the art in understanding the detailed description.

As used herein, the terms "non-compliant" and "semi-compliant" refer to an ability not to expand beyond a predetermined size or pressure and to substantially maintain a particular profile. Both non-compliant and semi-compliant balloons are able to expand to a specific size range under a particular pressure.

As used herein, the term "stent" refers to any support that can be inserted into a subject, such as for a purpose of improving healing or relieving an obstruction. Such a stent may, for example, be inserted into a lumen of a vessel so as to keep a passageway open.

As used herein, the term "lumen" refers to a cavity or channel within a tube or tubular organ, including but not limited to a blood vessel.

As used herein, the term "fibrotic tissue" refers to a tissue that is composed of bundles of fibers. Such fibrotic tissue may include but is not limited to webbing and scar tissue in the body.

As used herein, the terms "braid" and "braided" refer to a structure that includes weaved or interlaced strands of material.

As used herein, the term "dilatation" refers to a procedure whereby a vessel (including but not limited to a blood vessel) or other opening is dilated or otherwise enlarged.

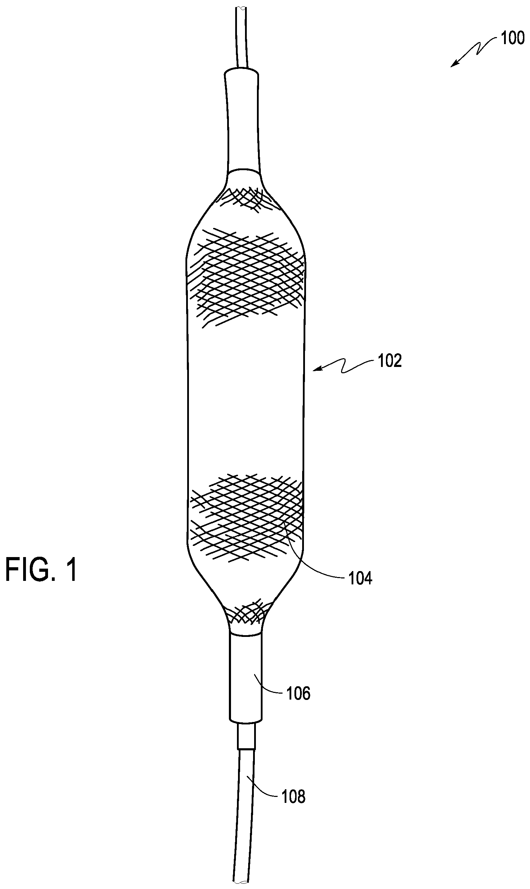

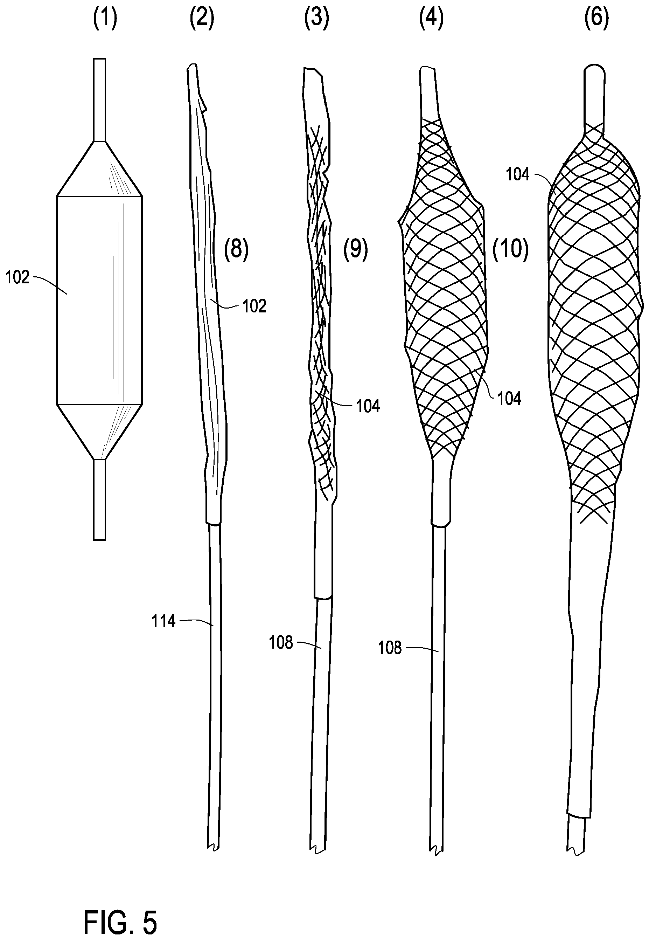

Referring to Figures 1-5, a balloon arrangement 100 is shown with Figure 5 showing different components of the balloon arrangement. The balloon arrangement 100 may be used, for example, to break up fibrotic tissue and create an open lumen prior to replacement of the stent. The balloon arrangement 100 allows a balloon to have a higher rated burst pressure than conventional balloon arrangements such that, for example, the balloon arrangement 100 can be pressurized to a level that allows for the breaking of fibrous webbing. Accordingly, in various embodiments, the balloon arrangement 100 may be used for the venous anatomy and venous application. However, it should be appreciated that the balloon arrangement 100 may be used for other anatomy and for different applications.

In some embodiments, the balloon arrangement 100 allow for opening up of a lumen prior to stenting. As a result, webbing/scar tissue that may impinge upon the stent and limit or even constrict its diameter may be reduced or eliminated. When using conventional balloons at these pressures, the balloons may rupture at the post-dilation stage, particularly if the fibrous webbing is hindering the stent expansion.

The balloon arrangement 100 includes a balloon 102 (such as a non-compliant or semi-compliant balloon) and an outer reinforcement structure 104 (such as an outer reinforced braided cage). In some embodiments, the balloon 102 defines a balloon layer and the outer reinforcement structure 104 defines a reinforcement layer.

The balloon 102 may be collapsible and expandable under pressure. The balloon 102 may be made of any suitable material. In some embodiments, the balloon layer may include a base layer. The base layer may be made of nylon, polyethylene terephthalate (PET), and/or a combination of the two, among others. The PET may be highly oriented PET.

The balloon 102 may include other layers, such as reinforcing layers, in some embodiment. In the embodiments shown in Figures 1-4, a balloon 102 with only a single base layer is shown. The balloon 102 in various embodiments has a wall thickness suitable to withstand high burst pressures reliably, including a pressure that allows for the breaking of fibrous webbing/scar tissue.

The outer reinforcement structure 104 in various embodiments is a braided cage that may be braided throughout its entire outer surface. Alternatively, the cage may be braided only in a portion of its surface. The outer reinforcement structure 104 may be made of any suitable material. In some embodiments, the outer reinforcement structure 104 is made, at least in part, of PET or similar monofilaments or multifilament material. Other suitable materials to be used in the cage may be Kevlar, Vectran, and UHMWPE, among others.

The outer reinforcement structure 104 may include a radiopaque braid, such as Pt-Ir or Pt-W. Such a radiopaque braid may be woven into the material. Owing to the features of the radiopaque braid, the outer reinforcement structure 104 and the balloon 102 may have improved visibility under fluoroscopy.

The orientation of the braided cage and particularities of the cage structure are not limited to described and illustrated configuration. While the Figures show an example embodiment of a braided cage with substantially even spacing between the braids, it will be understood that any cage with braids of any structure are within this disclosure. In some embodiments, the cage is braided so as to form substantially diamond spaces along the outer surface of the cage. The length dimension and width dimension of the diamond spaces are not particularly limited.

An expanded diameter of the balloon arrangement 100 (including at least the cage and the balloon 102) may be from about 10 to about 20 mm, or from about 12 to about 16 mm. In some embodiments, the expanded diameter of the balloon arrangement 100 may be 12, 14 or 16 mm. A longitudinal length of the balloon arrangement 100 (including at least the cage and the balloon 100) may be from about 50 to about 150 mm, or from about 60 to about 120 mm. In some embodiments, the length of the balloon arrangement 100 may be 60, 90 or 120 mm. However, different diameters and lengths may be provided as desired or needed.

The outer reinforcement structure 104 may be disposed around an outside of the balloon 102 so as to enclose an outer circumference of the balloon 102. According to the invention, ends of the outer reinforcement structure 104 are closed and/or coupled to other components by connecting components 106. The connecting components 106 connect a portion of the balloon 102 to a portion of the outer reinforcement structure 104.

An outer shaft 108 may be disposed so as to extend along and through the longitudinal axis of one or more of the balloon 102, outer reinforcement structure 104, and connectors 106. Thus, for example, at least a portion of the balloon 102 and a portion of the outer reinforcement structure 104 are disposed about and on the outer shaft 108.

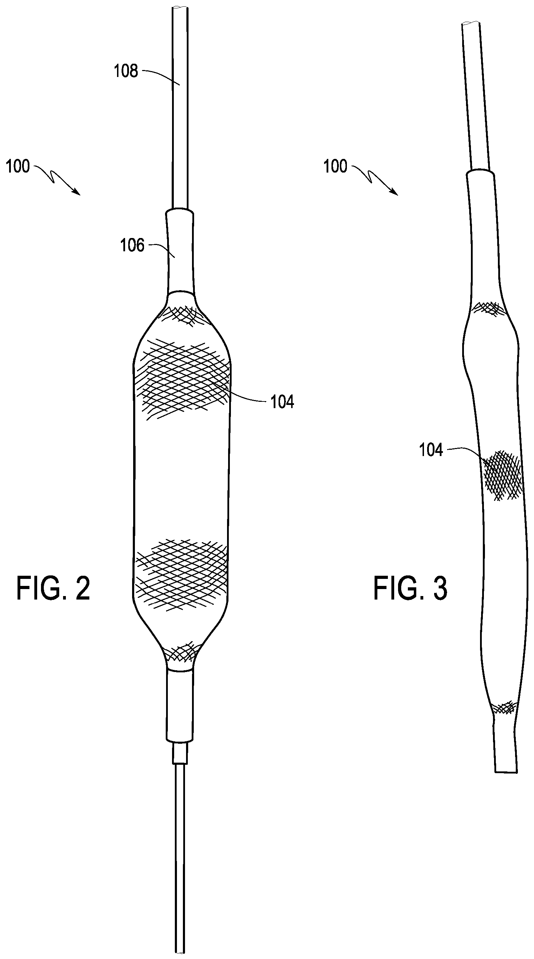

Figure 2 shows an expanded state of the balloon arrangement 100 including an expanded or pressurized state or position of the balloon 102 and outer reinforcement structure 104. Figure 3 shows a collapsed state of the balloon arrangement 100 including a collapsed or depressurized state or position of the balloon 102 and outer reinforcement structure 104.

Comparing Figures 2 and 3, the ratio of the expanded balloon arrangement 100 to the collapsed balloon arrangement 100 may be any suitable ratio. In some embodiments, the ratio is about 2:1 to 6:1, or about 4:1. However, the balloon 102 and outer reinforcement structure 104 may be configured to expand to various extents as desired or needed. In various embodiments, the outer reinforcement structure 104 allows the balloon 102 to expand to a desired or required pressure to allow, for example, to break up fibrotic tissue. Thus, in some embodiments, the outer reinforcement structure 104 allows the balloon 102 to expand to higher pressures that otherwise possible with conventional balloons and also may provide a protective cover over the balloon 102 to protect the balloon 102 and facilitate the breaking up of fibrotic tissue. The outer reinforcement structure 104 in some embodiments reduces the likelihood that the balloon 102 bursts at the higher pressures. As should be appreciated, the outer reinforcement structure 104 allows for higher pressure expansion of the balloon 102 for many different applications and the breaking up of fibrotic tissue is just one example.

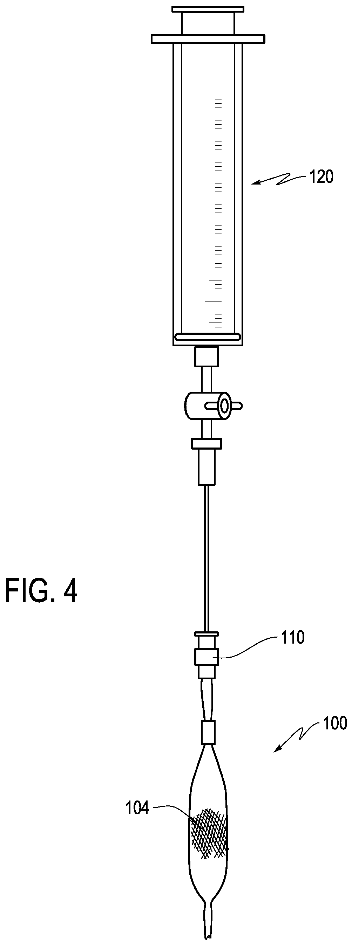

Figure 4 shows the balloon arrangement 100 of one embodiment and an injector device 120 coupled thereto for injecting and expanding the balloon 102 within a body, such as a lumen. In some embodiments, the balloon arrangement 100 will be injected into a human body. As can been seen in Figure 4, a coupling device 110 may be provided in combination with the balloon arrangement 100. For example, the coupling device 110 may be a hub, touhy-borst value or other suitable mechanism for coupling (e.g., removably coupling) the balloon arrangement 100 to the injector device 120.

Figure 5 illustrates different components of the balloon arrangement 100 that be provided together or separately as described in more detail herein. For example, one or more of the components by provided separately or with one or more other components to an end user. In some embodiments, one or more components of the balloon arrangement 100 not including the balloon 102 are provided and configured for attachment or coupling to a different balloon.

As shown in Figure 5, the balloon arrangement 100 includes the balloon 102 (shown in an expanded state and removed from the outer reinforcement structure 104 at (1)). As can be seen, the balloon 102 may be disposed on an inflation shaft 114, wherein (2) illustrates a folded or unexpanded or unpressurized balloon 104 coupled with the inflation shaft 114 at (8). The outer reinforcement structure 104, illustrated as a braided cage is shown at (3) in an unexpanded state at (9) coupled to the outer shaft 108. It should be appreciated that at the end of the outer shaft 108 opposite to the end at which the balloon 102 and outer reinforcement structure 104 are coupled the coupling device 110 may be provided, such as a hub to lock the balloon arrangement 100 to another device, such as a device that a physician would use to operate the balloon arrangement 100. For example, the coupling device 110 may be configured to close down on or couple with what is positioned within the coupling device 110.

At (4), an expanded braided at cage is shown at (10) separate from the outer shaft 108 shown therebelow at (5). The expanded braided cage is shown coupled with the outer shaft 108 and over the balloon 102 at (6). It should be noted that the outer reinforcement structure 104, such as the illustrated braided cage is an independent structure from the balloon 102 such that the outer reinforcement structure 104 is not part of the balloon structure. Thus, in various embodiments, the outer reinforcement structure 104 is a separate component from the balloon component. For example, the outer reinforcement structure 104 is movable and expandable separate from the balloon 102, but the outer reinforcement structure 104 and balloon 102 may both expand when the balloon 102 is expanded and applies pressure to the inner surface of the outer reinforcement structure 104. It should be noted that one or more components may be provided separate from or in combination with the other components. For example, in some embodiments, the cage/sleeve, outer shaft, and optionally hub/touhy-borst are provided. The assembly of these components may be used in conjunction with a standalone standard balloon catheter and make it capable of reaching higher pressures. This is these embodiments, the balloon 102 is separately provided.

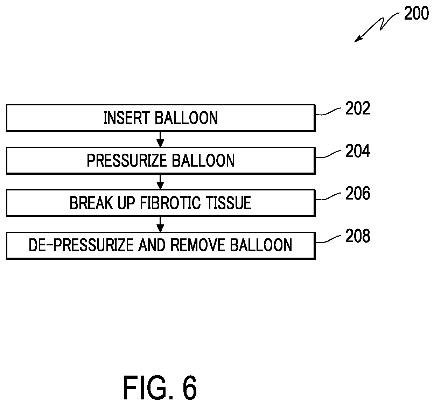

Figure 6 illustrates a method 200 for treating tissue that includes inserting a balloon, such as part of the balloon arrangement 100 into a subject having the tissue at 202. At 204, the balloon is pressurized such that the balloon arrangement 100 is pressurized so as to expand a diameter of the balloon arrangement 100, such as expanding the balloon 102 and outer reinforcement structure 104. The balloon arrangement 100 allows the balloon 102 to be pressurized to a higher pressure as described in more detail herein. The expanded balloon arrangement may be used to break up fibrotic tissue at 206. The outer reinforcement structure 104 then is de-pressurized and removed from the subject at 208. It should be noted that the balloon structure can include a pullback of the cage after the balloon inflation, which will help improve a deflation time and reduce the profile of the device. This may advantageously improve the efficiency of the removal of the balloon.

After the balloon is removed at 208, a stent may be introduced into the subject. While the stent may be introduced in any location, in various embodiments it will be applied to the location having the fibrotic tissue cleared or otherwise addressed by the balloon arrangement described herein. The use of the balloon may allow, for example, for the lumen of a vessel to be opened prior to the stenting, and the unwanted fibrotic tissue that would be present at such a location can be eliminated. Thus, the stent can then be applied at such a location whereby the location is free of restrictions and the stent can appropriately expand to its desired profile without delay and without any impinging by webbing or scar fibrotic tissue.

The amount of pressure that is provided to the balloon arrangement during its expansion may be substantial enough to be able to break up any fibrotic tissue such as scarring and/or webbing that may be necessary to move or remove. In a stenting procedure, the insertion of the balloon arrangement and subsequent addition of pressure may be used as a pre-dilatation procedure, so that the fibrotic tissue can be broken by the balloon arrangement and subsequently cleared prior to insertion of the stent. However, the balloon arrangement may also be suitably used as a post-dilatation procedure so as to allow for the inserted stent to achieve a larger diameter.

The balloon arrangement described herein may have a burst pressure high enough to break fibrotic material (webbing and scar tissue and the like) without losing desirable expansion and contraction characteristics. Further, the arrangement may be particularly suitably for the venous anatomy in view of its structure and composition. The braided cage described herein may allow, for example, for a radial and longitudinal constraint of the balloon so that there exists a physical barrier for the balloon to continue to expand. By limiting the expansion beyond that necessary to succeed in achieving a desired goal, failure of the balloon will be reduced or minimized, as the balloon material will be prevented from thinning or stretching, and failure locations such as pin holes, breaks, tears and ruptures are less likely to be seen. Further, the texture and orientation of the braided cage itself may aid in clearing and/or moving unwanted fibrotic tissue.

One skilled in the art would appreciate that each of the embodiments described above are not mutually exclusive. One skilled in the art would recognize that the medical balloon structure provided herein may be in medical applications beyond stenting procedures, so long as a desire or need for enlarging a space exists.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the various embodiments without departing from their scope. While the dimensions and types of materials described herein are intended to define the parameters of the various embodiments, the embodiments are by no means limiting and are exemplary embodiments. Many other embodiments will be apparent to those of skill in the art upon reviewing the above description. The scope of the various embodiments should, therefore, be determined with reference to the appended claims. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects.

This written description uses examples to disclose the various embodiments, including the best mode, and also to enable any person skilled in the art to practice the various embodiments, including making and using any devices or systems. The patentable scope of the various embodiments is defined by the claims, and may include other examples that occur to those skilled in the art. Such other examples are intended to be within the scope of the claims if the examples have structural elements that do not differ from the literal language of the claims.

## Claims

1. A balloon arrangement (100), comprising:
a balloon (102) comprising a base layer formed of polymer material; and
an outer reinforcement structure (104) independent from the balloon (102), the outer reinforcement structure (104) comprising a monofilament and/or a multifilament material, and adapted to come into contact with the base layer during pressurization,
wherein
the outer reinforcement structure (104) is a braided cage;
the outer reinforcement structure (104) is a separate component from the balloon (102) so that the outer reinforcement structure (104) is movable and expandable separate from the balloon (102) but the outer reinforcement structure (104) and balloon (102) both expand when the balloon (102) is expanded and applies pressure to an inner surface of the outer reinforcement structure (104); **characterized in that**
the balloon arrangement further comprises a plurality of connector portions (106) disposed at both longitudinal ends of the braided cage and configured to couple the longitudinal ends of the balloon (102) to the respective longitudinal ends of the braided cage.

2. The balloon arrangement according to claim 1, wherein a diameter of the balloon arrangement (100) is about 12 to about 16 mm.

3. The balloon arrangement according to claim 1, wherein a length of the balloon arrangement is about 60 to about 120 mm.

4. The balloon arrangement according to claim 1, further comprising an outer shaft (108), wherein the outer shaft (108) is disposed through a central portion of the balloon (102) and braided cage, and extends along a longitudinal direction of the balloon and braided cage.

5. The balloon arrangement according to claim 1, wherein the braided cage comprises at least one of Kevlar, Vectran and UHMWPE.

6. The balloon arrangement according to claim 1, wherein the braided cage is composed of radiopaque material.

7. The balloon arrangement according to claim 1, wherein the balloon (102) is non-compliant.

8. The balloon arrangement according to claim 1, wherein the balloon (102) is semi-compliant.

9. The balloon arrangement according to claim 1, wherein the braided cage is comprised of expandable material.

10. The balloon arrangement according to claim 9, wherein a ratio of a collapsed diameter of the balloon arrangement to an expanded diameter of the balloon arrangement is about 4:1.

11. The balloon arrangement according to claim 9, wherein, during expansion, the diameter of the braided cage increases commensurately with an expanding diameter of the balloon (102).

## Patentansprüche

1. Ballonanordnung (100), aufweisend:
einen Ballon (102), der eine Basisschicht aufweist, die aus einem Polymermaterial ausgebildet ist; und
eine äußere Verstärkungsstruktur (104), die unabhängig von dem Ballon (102) ist, wobei die äußere Verstärkungsstruktur (104) ein Monofilament- und/oder ein Multifilament-Material aufweist und dazu eingerichtet ist, während einer Druckbeaufschlagung mit der Basisschicht in Kontakt zu treten,
wobei
die äußere Verstärkungsstruktur (104) ein geflochtener Käfig ist;
die äußere Verstärkungsstruktur (104) eine von dem Ballon (102) getrennte Komponente ist, so dass die äußere Verstärkungsstruktur (104) getrennt von dem Ballon (102) bewegbar und expandierbar ist, jedoch die äußere Verstärkungsstruktur (104) und der Ballon (102) beide expandieren, wenn der Ballon (102) expandiert wird und Druck auf eine Innenfläche der äußeren Verstärkungsstruktur (104) ausübt;
**dadurch gekennzeichnet, dass**
die Ballonanordnung zusätzlich mehrere Verbindungsabschnitte (106) aufweist, die an beiden longitudinalen Enden des geflochtenen Käfigs angeordnet und dazu eingerichtet sind, die longitudinalen Enden des Ballons (102) mit den jeweiligen longitudinalen Enden des geflochtenen Käfigs zu koppeln.

2. Ballonanordnung nach Anspruch 1, wobei ein Durchmesser der Ballonanordnung (100) etwa 12 bis etwa 16 mm beträgt.

3. Ballonanordnung nach Anspruch 1, wobei eine Länge der Ballonanordnung etwa 60 bis etwa 120 mm beträgt.

4. Ballonanordnung nach Anspruch 1, die zusätzlich einen äußeren Schaft (108) aufweist, wobei der äußere Schaft (108) durch einen zentralen Abschnitt des Ballons (102) und des geflochtenen Käfigs angeordnet ist und sich entlang einer Längsrichtung des Ballons und des geflochtenen Käfigs erstreckt.

5. Ballonanordnung nach Anspruch 1, wobei der geflochtene Käfig mindestens eines von Kevlar, Vectran und UHMWPE aufweist.

6. Ballonanordnung nach Anspruch 1, wobei der geflochtene Käfig aus röntgendichtem Material aufgebaut ist.

7. Ballonanordnung nach Anspruch 1, wobei der Ballon (102) nicht nachgiebig ist.

8. Ballonanordnung nach Anspruch 1, wobei der Ballon (102) teilnachgiebig ist.

9. Ballonanordnung nach Anspruch 1, wobei der geflochtene Käfig aus einem expandierbaren Material aufgebaut ist.

10. Ballonanordnung nach Anspruch 9, wobei ein Verhältnis eines zusammengefalteten Durchmessers der Ballonanordnung zu einem expandierten Durchmesser der Ballonanordnung etwa 4:1 beträgt.

11. Ballonanordnung nach Anspruch 9, wobei der Durchmesser des geflochtenen Käfigs während der Expansion entsprechend einem expandierenden Durchmesser des Ballons (102) zunimmt.

## Revendications

1. Agencement de ballonnet (100), comprenant :
un ballonnet (102) comprenant une couche de base formée d'un matériau polymère ; et
une structure de renforcement extérieure (104) indépendante du ballonnet (102), la structure de renforcement extérieure (104) comprenant un matériau monofilament et/ou multifilament et étant adaptée pour entrer en contact avec la couche de base lors d'une mise sous pression,
dans lequel
la structure de renforcement extérieure (104) est une cage tressée ;
la structure de renforcement extérieure (104) est un composant séparé du ballonnet (102) de sorte que la structure de renforcement extérieure (104) soit mobile et qu'elle se dilate séparément du ballonnet (102) mais que la structure de renforcement extérieure (104) et le ballonnet (102) se dilatent tous deux lorsque le ballonnet (102) est dilaté et qu'il applique une pression sur une surface intérieure de la structure de renforcement extérieure (104) ; **caractérisé en ce que**
l'agencement de ballonnet comprend en outre une pluralité de parties de connecteur (106) disposées aux deux extrémités longitudinales de la cage tressée et configurées pour coupler les extrémités longitudinales du ballonnet (102) aux extrémités longitudinales respectives de la cage tressée.

2. Agencement de ballonnet selon la revendication 1, dans lequel un diamètre de l'agencement de ballonnet (100) est d'environ 12 mm à environ 16 mm.

3. Agencement de ballonnet selon la revendication 1, dans lequel un longueur de l'agencement de ballonnet est d'environ 60 mm à environ 120 mm.

4. Agencement de ballonnet selon la revendication 1, comprenant en outre un arbre extérieur (108), dans lequel l'arbre extérieur (108) est disposé à travers une partie centrale du ballonnet (102) et de la cage tressée, et s'étend sur une direction longitudinale du ballonnet et de la cage tressée.

5. Agencement de ballonnet selon la revendication 1, dans lequel la cage tressée comprend au moins un parmi Kevlar, Vectran et UHMWPE.

6. Agencement de ballonnet selon la revendication 1, dans lequel la cage tressée est composée d'un matériau radio-opaque.

7. Agencement de ballonnet selon la revendication 1, dans lequel le ballonnet (102) est non-élastique.

8. Agencement de ballonnet selon la revendication 1, dans lequel le ballonnet (102) est semi-élastique.

9. Agencement de ballonnet selon la revendication 1, dans lequel la cage tressée est constituée d'un matériau dilatable.

10. Agencement de ballonnet selon la revendication 9, dans lequel un rapport d'un diamètre de repli de l'agencement de ballonnet sur un diamètre de dilatation de l'agencement de ballonnet est d'environ 4:1.

11. Agencement de ballonnet selon la revendication 9, dans lequel, lors de la dilatation, le diamètre de la cage tressée augmente de manière proportionnée à un diamètre de dilatation du ballonnet (102).
